# EUROPEAN PATENT SPECIFICATION

(11) **EP 4 232 821 B1**
(45) Date of publication and mention of the grant of the patent: **23.07.2025**
(21) Application number: 21801842.2
(22) Date of filing: 19.10.2021
(51) Int. Cl.: G01N 33/574, G01N 27/00

(54) **DNA DAMAGE SENSITIVITY AS A NEW BIOMARKER FOR DETECTION OF CANCER AND CANCER SUSCEPTIBILITY**
DNA-SCHADENSEMPFINDLICHKEIT ALS NEUER BIOMARKER ZUM NACHWEIS VON KREBS UND KREBSEMPFINDLICHKEIT
SENSIBILITÉ À L'ENDOMMAGEMENT DE L'ADN EN TANT QUE NOUVEAU BIOMARQUEUR POUR LA DÉTECTION DU CANCER ET DE LA PRÉDISPOSITION AU CANCER

(30) Priority: 22.10.2020 CH 13632020
(43) Date of publication of application: 30.08.2023
(73) Proprietor: 4D Lifetec AG, 6330 Cham (CH)
(72) Inventor: FAISST, Arne, 6330 Cham (CH); GODAU, Julia, 6330 Cham (CH); SCHICHT, Oliver, 6330 Cham (CH)
(74) Representative: Frei Patent Attorneys
(86) International application number: PCT/EP2021/078953
(87) International publication number: WO 2022/084318

(56) References cited:
- EP-A1- 3 508 841
- FARIVAR NEGIN: "UV-Induced DNA damage response in blood cells as a potential method for cancer detection", 1 April 2019 (2019-04-01), pages 1 - 82, XP055881102, Retrieved from the Internet <URL:https://open.library.ubc.ca/soa/cIRcle/collections/ubctheses/24/items/1.0378307> [retrieved on 20220119]
- GABRIELA FIGUEROA-GONZÁLEZ ET AL: "Strategies for the evaluation of DNA damage and repair mechanisms in cancer", ONCOLOGY LETTERS, vol. 13, no. 6, 6 April 2017 (2017-04-06), GR, pages 3982 - 3988, XP055648535, ISSN: 1792-1074, DOI: 10.3892/ol.2017.6002
- MCKENNA DECLAN J. ET AL: "Use of the Comet-FISH Assay to Compare DNA Damage and Repair in p53 and hTERT Genes following Ionizing Radiation", PLOS ONE, vol. 7, no. 11, 7 November 2012 (2012-11-07), pages e49364, XP055873920, Retrieved from the Internet <URL:https://journals.plos.org/plosone/article/file?id=10.1371/journal.pone.0049364&type=printable> DOI: 10.1371/journal.pone.0049364
- FARIVAR NEGIN ET AL: "UV-induced DNA damage response in blood cells for cancer detection", MEDICAL DEVICES & SENSORS, vol. 4, no. 1, 26 November 2020 (2020-11-26), XP055881095, ISSN: 2573-802X, Retrieved from the Internet <URL:https://onlinelibrary.wiley.com/doi/full-xml/10.1002/mds3.10146> DOI: 10.1002/mds3.10146

## Description

### FIELD OF THE INVENTION

The invention relates to a marker that can be used as biomarker for detection of cancer. More specifically, the present invention relates to the analysis of genomic DNA damage sensitivity (DDS) as a biomarker for cancer or cancer susceptibility or other disease states characterized by DNA damage response (DDR) disturbances. The biomarker of the invention can also be used to study the effect of medication, risk factors and/or environmental impacts on the susceptibility to cancer or more generally to risk factors that impair genomic DNA damage sensitivity (DDS).

### BACKGROUND OF THE INVENTION

The diagnostic and prognostic role of DNA damage sensitivity has recently been investigated. Within the present invention, it was possible to implement a method for determining genomic DNA damage sensitivity (DDS) as a marker for a proliferative disease. The method is based on single cell gel electrophoresis (SCGE).

SCGE, also called comet assay, is a sensitive method for the direct visualization and quantification of damaged DNA, such as DNA single-strand breaks and double-strand breaks. The cells, which are to be examined or treated, can be embedded e.g., into agarose, and can be deposited as so-called gel spots onto a carrier material such as for example onto an object carrier or film. Those embedded cells are then lysed and either treated in an alkaline manner in order to denature the DNA or alternatively kept in a neutral environment to remove the cell membranes and most of the proteins. Both ways result in supercoiled DNA linked to the nuclear matrix; a structure sometimes referred to as nucleoids. The subsequent electrophoresis leads to damaged DNA removing itself from the nucleoid when exposed of an electrical field, i.e., the negatively charged DNA fragments travel to the plus pole and produce a so-called "comet". The amount of DNA, which has migrated out of the head of the comet into the tail is quantified and serves as a quantitative measure of the DNA damage present in the sample. The successful quantification of the comet tail is mainly affected by (i) the performance of the gel-electrophoresis system and (ii) the DNA visualization and quantification method e.g., the type of fluorescence microscopy and imaging software, and is implemented in a manual, partially automated or fully automated manner. High-performing gel electrophoresis systems are known e.g., from WO 2016/141 495. Single cell gel electrophoresis devices and methods suitable for screening a subject for DNA damage with a high reproducibility are known from WO 2019/135 008 and EP 3508841 A1 respectively.

Gabriela Fugueroa-Gonzalez and Carlos Perez-Plasencia (Oncology Letters 13: 3982-3988, 2017) summarized differend strategies for the evaluation of DNA damage and repair mechanism in cancer wherea one method described is the Comet assay. McKenna Declan et al (PLOS ONE, vol. 7, no. 11, page e49364 described in 2012 the use of a combination of the Comet Assay and FISH methodology to localize specific gene domains within an individual cell.

The underlying biological principle is that genomic DNA of healthy individuals and cancer patients reacts differently to radiation such as electromagnetic radiation. Farivar Negin evaluated and compared in its master thesis 2019 the degree of UV-induced DNA damage between cancer patients and healthy individuals analysing yH2AX expression. They concluded from their results that the "expression of yH2AX which is an indicator of DNA double strand breaks and the degree of the UV-induced DNA damage was higher in PBMCs isolated from cancer patient blood samples."

A correlation between DNA sensitivity to damage by radiation and the susceptibility of a person to cancer has been described in WO 2014/041340 A1. The main difference is that the inventors could show that the correlation is reverse. Using the method of the present invention it could be shown that healthy surrogate cells, such as e.g., Peripheral Blood Mononuclear Cells (PBMCs), from healthy subjects have a higher sensitivity to electromagnetic radiation compared to healthy surrogate cells e.g. PBMCs of cancer patients. This is not known so far. It has been proposed that during tumorigenesis, the chromosomal landscape of cells is changing. It influences the chromatin structure in various ways including DNA hyper- or hypomethylation, DNA accessibility and transcription-bound factors or histone modifications (Zhao et al. 2020; Mao et al. 2016; for review: Brock et al. 2007; McAnena et al. 2016). But it was not described that the genomic DNA becomes less sensitive to radiation.

### SUMMARY OF THE INVENTION

The objective of the present invention is to provide biomarkers for an early and highly sensitive detection of cancer with strong predictive power. This goal is achieved by detection of DNA damage resistance against radiation and the insight that using the methods of the invention, genomic DNA of cells from healthy controls is more sensitive than genomic DNA of cells from cancer patients of all cancer stages or individuals having an increased risk for cancer.

The invention refers particularly to an *ex vivo* or *in vitro* method related to determining resistance of genomic DNA against damage by radiation
comprising:
a) providing a sample containing blood cells,
b) irradiating the blood cells of the sample,
c) performing cell lysis, and
d) determining genomic DNA damage using single cell gel electrophoresis assay,
wherein an increased resistance of the genomic DNA is indicative for a proliferative disease and/or increased susceptibility for a proliferative disease.. The present invention refers to an *ex vivo* or *in vitro* use of genomic DNA damage sensitivity (induced by radiation) as a biomarker for proliferative diseases and susceptibility thereof, wherein decreased genomic DNA damage sensitivity is indicative for a proliferative disease and/or increased susceptibility thereof. The ex vivo or in vitro method comprises analyzing a biological sample containing cells from a subject to determine the resistance of genomic DNA. The methods of the present invention are suitable for cancer screening, early diagnosis (of early stages of the cancer) of cancer, monitoring for recurrence of cancer after treatment and remission. Most cancers that involve a tumor are staged in five broad groups, stage 0 to IV. It could be shown that even very early stages (such as stage 0 and/or I) can be detected using the present invention.

Cancer stage refers to the extent of a cancer, such as how large the tumor is, and if it has spread. The extent is often primarily assessed on the basis of size and its localization, but other factors such as a histopathological assessment also play an important role in the overall assessment of a tumor disease. Knowledge of a tumor "stage" is critical for treatment planning and prognosis in malignant tumor disease. The UICC (Union internationale contre le cancer) TNM Classification of Malignant Tumors (TNM) is a globally recognized standard for classifying the extent of spread of cancer. The T category describes the primary tumor site and size, the N category describes the regional lymph node involvement, and the M category describes the presence or otherwise of distant metastatic spread.

The biomarker may also be used to monitor the response to a treatment of cancer or to investigate the likeliness to response (responsiveness) to a specific treatment option in order to find the best treatment strategy. Another use may be studies concerning the effect of medication, risk factors and/or environmental impacts on the susceptibility to cancer or more generally to risk factors that impair genomic DNA damage sensitivity (DDS). Therefore, cells, bacteria, yeast, protozoa, plants or animals may be brought into contact with a candidate substance (possible active agent, risk factor). Thereafter the method according to the invention is used to screen if the candidate substance has influence on genomic DNA damage sensitivity (DDS) and on cancer susceptibility, cancer progress or treatment of proliferative diseases.

The term "proliferative disease" refers herein to tumors, in particular solid tumors, cancer, malignancies and their metastasis. Examples for proliferative diseases are adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gallbladder carcinomas, uterine cancer, cervical cancer, cervix, glioblastomas, gynecologic tumors, ear, nose and throat tumors, hematologic neoplasia, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and an lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarian carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer. Cancer as used herein refers to diseases involving abnormal cell growth with the potential to invade or spread to other parts of the body.

Cancers may be classified by the type of cell that is presumed to be the origin of the tumor. The *ex vivo* or *in vitro* method according to the present invention relates therefore to determining resistance of genomic DNA against damage by radiation as a biomarker for proliferative diseases such as carcinoma (derived from epithelial cells), sarcoma (arising from connective tissue or respectively cells originating in mesenchymal cells outside the bone marrow), lymphoma and leukemia (arising from hematopoietic cells), germ cell tumour, and blastoma (derived from immature "precursor" cells or embryonic tissue).

The group of carcinoma includes among others breast cancer, prostate cancer, lung cancer, pancreas cancer and colon cancer. The group of sarcoma includes among others bone cancer and cancer derived from cartilage, fat, and nerve cells. The proliferative disease, the present invention relates to, may in particular be selected from the group consisting of: Bladder cancer, breast cancer, colon and rectal cancer, endometrial cancer, kidney cancer, leukemia, liver cancer, lung cancer, melanoma, non-Hodgkin's lymphoma, pancreatic cancer, prostate cancer, and thyroid cancer. The biomarker as described herein is preferably used for detection of solid tumors and in particular breast cancer, lung cancer, prostate cancer, colon cancer or an increased susceptibility for one of these cancers. Breast cancer comprises non-invasive and invasive ductal carcinoma as well as occult invasive breast cancer. The term "lung cancer" comprises small cell lung cancer (SCLC) and non-small cell lung cancer (NSCLC). The colorectal cancer and prostate cancer refer mainly to adenocarcinoma within the bowel, the colon, the rectum or respectively the prostate.

Determining the sensitivity of genomic DNA against damage might be helpful for screening and monitoring human malignancies. Today it is possible to identify persons having a higher risk for cancer, for example, because of familiar events, genetic predisposition (genetic variations, genetic defects) or environmental impacts (such as exposure to toxins or smoking). The present invention enables to monitor these people with a simple test. Monitoring the response to cancer treatment may be another possible application for the present invention because the biomarker can also be used to monitor in a non-invasive way the recurrence of cancer after successful anti-cancer treatment and remission. One goal of the present invention is also a follow-up care which means to check for a recurrence of a cancer after successful treatment.

The *ex vivo* or *in vitro* method can be related to determining resistance of genomic DNA against damage by radiation as a biomarker for proliferative diseases and a susceptibility for proliferative diseases, wherein the genomic DNA is derived from healthy surrogate cells of the patient. The sample is not obtained from the cancer as such. It is preferred that the genomic DNA is derived from whole blood cells or from peripheral blood cells containing e.g., PBMCs or more specifically mainly lymphocytes. The cell concentration in the sample can be approximately 200-400 cells per spot. Within the present invention, it is preferred that one sample represents one spot within the agarose gel. For controls and for doubletted measurements, it may be suitable to collect more blood per individual and divide this blood specimen into several samples within the method according to the invention.

The radiation used to introduce DNA damage within the genomic DNA may be UV-light and more preferred UVB radiation. The electromagnetic spectrum of ultraviolet radiation, defined most broadly as 10-400 nanometers, can be subdivided into a number of ranges. One is UVB which is an intermediate range with a wavelength of 280-315 nm.

The *ex vivo* or *in vitro* method related to determining resistance of genomic DNA against damage by radiation as a biomarker for proliferative diseases and a susceptibility for proliferative diseases may be suitable to test the effect of chemical compounds, medication, and/or environmental impacts on cancer susceptibility.

There are different methods known to quantitatively determine DNA damage and most of them analyze either tumor DNA obtained from the tumor or circulating tumor DNA (ctDNA) from the peripheral blood. These methods are most often based on detecting sequence alterations at the molecular level in the tumor DNA. These can suffer from low sensitivity when either circulating tumor cells or ctDNA need to be captured from the blood for sequencing. A completely different approach is used here, by collecting living blood cells such as PBMC, challenging them, and finally assaying them in a single cell gel electrophoresis (also called comet assay) assessing the subsequent determination of the DNA-damage. An automated analysis can be performed. This strengthens the meaningfulness and ensures the performance, interoperability and schedulability of the single cell gel electrophoresis ("comet assay") test. The present method may be combined with determination of at least one further biomarker for cancer. The use of resistance of genomic DNA against damage by radiation as a biomarker for proliferative diseases and susceptibility thereof can involve that the DNA damage is determined as normalized DNA tail using single cell gel electrophoresis assay. Such an assay may be performed as follows: A liquid sample is applied on a carrier plate, mostly within an agarose gel. The sample may be irradiated before it is introduced into the agarose gel. However, it is preferred that the sample is exposed to radiation after introduction into the agarose gel. Thereafter the cells within the sample are lysed. The carrier plate is positioned in a homogenous electrical field generated by at least one pair of electrodes of a gel electrophoresis device. By controlling strength and direction of the electrical field generated at the position of the samples accommodated on the carrier plate a single cell gel electrophoresis (SCGE) is performed. The single nucleoids are depicted as whitish dots also called comets (the color of the dots may vary depending on the staining or imaging). The single comets, each representing a single cell, show a head on the one side and a tail on the other side. The orientation of the comet depends on the orientation of the single cells in the electrical field. From the ratio between the tail and the head of the comet/single cell, the DNA damage can be determined.

The present invention refers to an *ex vivo* or *in vitro* method for determining the resistance of genomic DNA against damage by radiation, comprising:
a) providing a sample containing blood cells,
b) irradiating the blood cells of the sample,
c) performing cell lysis, and
d) determining genomic DNA damage using single cell gel electrophoresis assay, wherein an increased resistance of the genomic DNA is indicative for a proliferative disease and/or increased susceptibility for a proliferative disease.

Within said method, an increased resistance of the genomic DNA is indicative for a proliferative disease and/or increased susceptibility for a proliferative disease. Whole blood samples or a sample containing isolated peripheral blood (mononuclear) cells are suitable for this method. It is most suitable to use a liquid sample, a so-called liquid biopsy.

The cells within the sample are irradiated. The radiation used may be UV radiation, preferably in the range of UVB. Before or after radiation, the cells of the sample are embedded in an agarose gel. Immediately after irradiation, the cells are lysed. Cell lysis can take place less than 10 minutes, preferably less than 7 minutes or less than 5 minutes and more preferred less than 3 minutes after irradiation of the blood cells. Cell lysis as used herein refers to opening of cells. Lysis may be affected by enzymes or detergents or other chaotropic agents. Mechanical disruption of cell membranes, as by repeated freezing and thawing, sonication, pressure, or filtration may also be used. However, it is desirable to avoid mechanical shear forces that would denature or degrade DNA.

It has been shown that electromagnetic radiation induces in cells of healthy persons more DNA damage than in respective cells of cancer patients. It is thereby very surprising that the cells of healthy test persons are more susceptible to damage than the cells of cancer patients. In the state of the art, an opposite effect is shown, for example, Anderson et al. 2014 (Anderson D, Najafzadeh M, Gopalan R, et al. Sensitivity and specificity of the empirical lymphocyte genome sensitivity (LGS) assay: implications for improving cancer diagnostics. FASEB J. 2014;28(10):4563-4570. doi:10.1096/fj.14-254748)). However, the results of the inventors show that genomic DNA of blood cells of cancer patients is better protected against UVB damage than cells from healthy individuals.

Currently, there is no definite scientific rational explanation for this difference. Different various hypotheses are highly likely. Cancer is based on pathological changes in the genome of tumor cells. It is quite possible that circulating healthy cells such as peripheral blood mononuclear cells (PBMCs) from patients receive a direct or indirect signal that causes these cells to protect their genome from DNA damage. Protection may occur by epigenetic changes and/or DNA and histone modifications or other post-translational changes. Based on this hypothesis it should make no difference at all in which organ of the body a cancer is located, since the cells of the circulating blood will at some stage receive this direct or indirect signal. This opens a large potential for the DDS biomarker to become a multiple-cancer or pan-cancer biomarker.

The reason for the difference could also be the time point of the measurement. To resolve DNA damage and maintain genome integrity, cells have developed a complex mechanism, collectively known as the DNA damage response (DDR) network. The DNA damage response network comprises a variety of cellular processes in which cells pause the cell cycle to allow time for repair, activate their DNA repair pathways, and stop replication to prevent replication of damaged DNA template or, if the lesion is too severe, trigger cell death. The DNA damage response network is essential for sensing, processing and repairing DNA damage and is frequently disrupted in cancer. It may be decisive whether DNA damage response mechanisms have already started and how effective they are in the respective cells. It may be that the healthy cells repair more efficiently, but that the DNA damage response mechanisms of PBMCs derived from healthy subjects are significantly different time-wise compared to cells derived from cancer patients leading to a measurable alteration in DNA damage sensitivity.

When using the methods described herein cancer is diagnosed in case that DNA damage is less compared to control values of healthy patients and cancer susceptibility is higher the less the DNA damage is. In other words, cancer is diagnosed in case that DNA damage sensitivity (DDS) is less compared to control values of healthy donors and cancer susceptibility is higher the less the DNA damage is. According to methods described herein a cancer diagnosis is made when the DTN as measure for DNA damage is decreased in the sample of an individual at least 1.05-fold, preferred at least 1.1-fold, more preferred at least 1.15-fold, more preferred at least 1.2-fold, more preferred at least 1.3-fold, more preferred at least 1.4-fold, more preferred at least 1.6-fold, more preferred at least 1.7-fold, more preferred at least 1.8-fold, more preferred at least 1.9-fold, more preferred at least 2-fold, more preferred at least 2.1-fold more preferred at least 2.2-fold, more preferred at least 2.3-fold, more preferred at least 2.4-fold, more preferred at least 2.5-fold, more preferred at least 2.6-fold, more preferred at least 2.7-fold, more preferred at least 2.8-fold, more preferred at least 2.9-fold, more preferred at least 3.0-fold, more preferred at least 3.1-fold, more preferred at least 3.2-fold, more preferred at least 3.3-fold, more preferred at least 3.4-fold, more preferred at least 3.5-fold, more preferred at least 3.6-fold, more preferred at least 3.7-fold, more preferred at least 3.8-fold, more preferred at least 3.9-fold, and even more preferred at least 4.0-fold compared to the DNA damage in samples from healthy donors.

For controls, to ascertain whether the method is properly working in the hands of the operator, a reference sample (a sample validated to be an intra and inter assay control) should always be used together with samples of interest (of individuals to be assayed for the detection of cancer). This is a common feature of biological or diagnostic assays. This means that at the same time or immediately one after the other, not only the sample of the person to be tested is assayed, but also a reference sample, which can be a blank and/or a sample derived from a healthy donor or a pool of samples from healthy donors.

Step d) "determining genomic DNA damage using single cell gel electrophoresis assay" of the method according to the present invention may include at least two sub steps, namely:
Performing single cell gel electrophoresis and analysis of the data obtained by single cell gel electrophoresis. An analysis of data obtained by single cell gel electrophoresis can be divided into image evaluation and calculation using data obtained by image evaluation.

The DNA damage is evaluated by acquiring photographs of the sample spot. It may be that the image of one spot is stitched together from several single shots. Possibly, only one single image of the spot can be required for determining the DNA damage. In other words: One shot of respectively image, namely one photo, is taken from one spot, namely one sample, comprising the single cells. Data gained from image analysis such as "DNA Tail Percent (DT%) or Olive Tail Moment (OTM)" which can be determined by known algorithm, may be used to compare the amount of DNA damage of samples and controls of healthy donors. The data analysis can be done by commercially available software such as Komet7^{™}, and CometIV^{™}.

Within the present invention it is preferred that step d) "determining genomic DNA damage using single cell gel electrophoresis assay" comprises comparison/evaluation of DNATail Normalized (DTN). DNATail Normalized (DTN) is calculated as DNA Tail (DT, percentage of the total light intensity measured for a comet in the tail) % at time point after UV exposure minus DT% in unperturbed condition (or control or baseline). Using Komet^{®} by ANDOR one may vary the results depending on the selection of Comet Options, and particularly with selection/deselection of the Head Symmetry option. The preferred calculation of (DT%) is based on %HeadDNA = (Head.OptInten /(Head.OptInten + Tail.OptInten))*100. OptInten= optical intensity in respective region 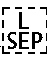 %Tail-DNA= 100-%HeadDNA.

It is possible to use rather simple algorithms. Thus, one may determine and compare the absolute difference between the measured values in healthy people and patients or respectively suspected cancer patients. However, it is also possible to use other algorithms, for example, based on relative differences or calibrating the individual experiment. Determining genomic DNA damage using a single cell gel electrophoresis assay may comprise the calculation and evaluation of DT% as one possible algorithm for how the data are analyzed.

### BRIEF DESCRIPTION OF THE DRAWINGS

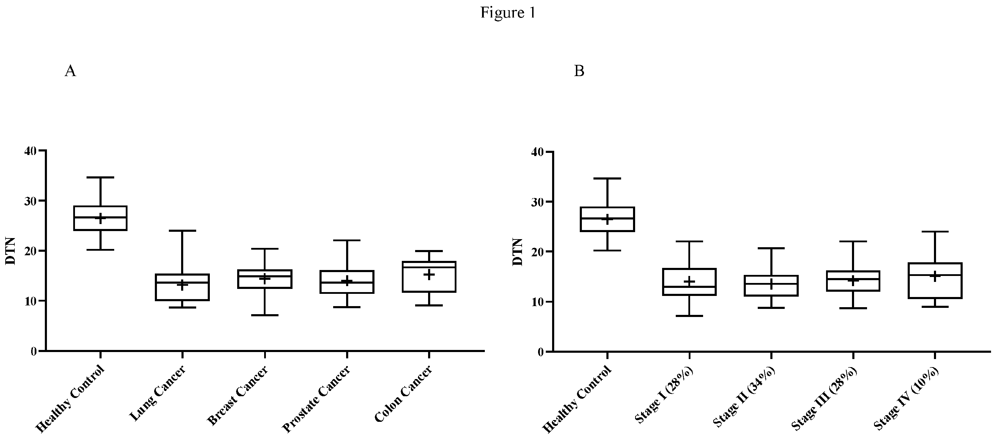

Figure 1 is a graphical representation of data collected using a method of the described invention. It illustrates the DTN (DT% after UVB exposure normalized with baseline values) of samples derived from 31 healthy donors compared to 19 breast cancer, 23 lung cancer, 42 prostate and 15 colon cancer patients. Box plots display the median (line inside the boxes) the 75 % percentile (top of the boxes), and the 25 % percentile (bottom of boxes) and whiskers represent the minimum and maximum of the values. The "+" indicates the mean. (A) Shows the DNA damage sensitivity determined by DTN from the four most prominent cancer types. (B) Same data as A but sorted by cancer stage if known. Stage 0 of breast cancer patients was included in stage I. Percentages describe the frequency in the sample population.

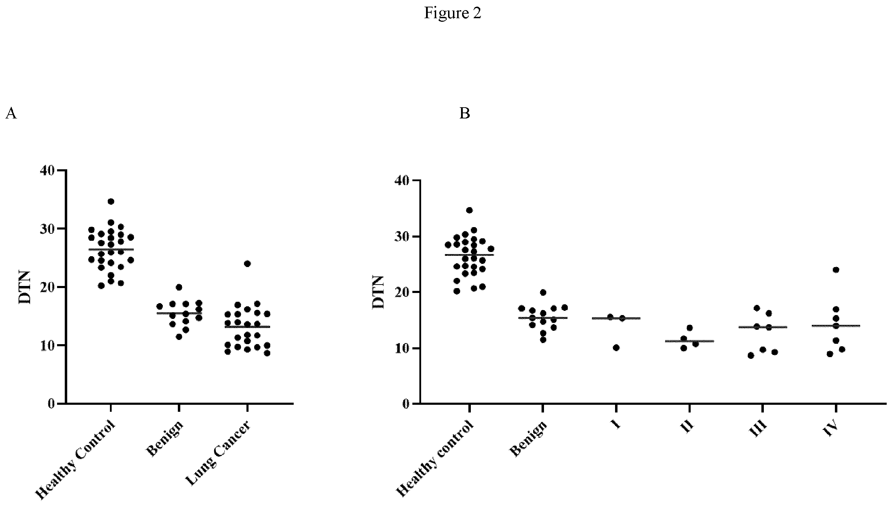

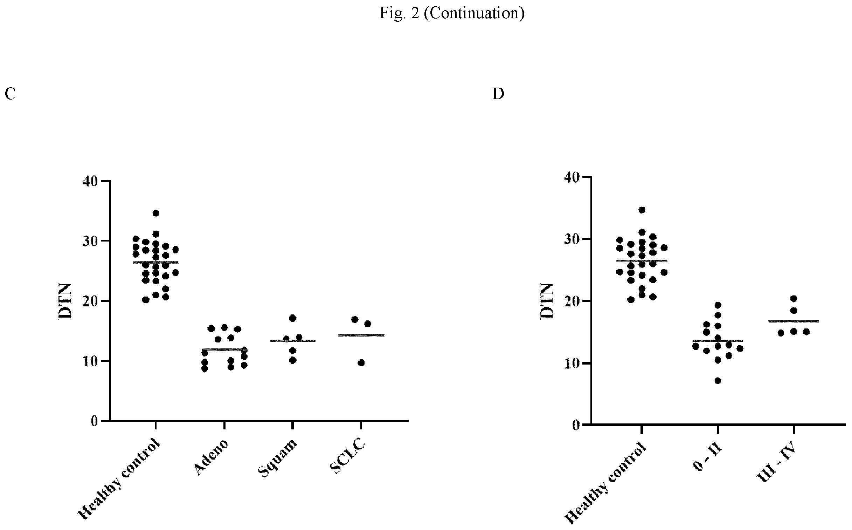

Figure 2 illustrates data using method of the described invention as DTN (%DNA tail after UVB exposure normalized with baseline values) values of samples derived from 31 healthy donors compared to 23 lung cancer and 13 benign tumor patients. Each dot represents a single individual subject. The horizontal line indicates the mean. (A) shows the susceptibility of lung cancer patients and benign cohort compared to healthy control group. (B) Same data as in A with lung cancer samples sorted by stage. (C) Same lung cancer data as in A grouped by lung cancer types if known. (D) Sensitivity of only breast cancer patient-derived samples sorted by stage.

Our data reveal the described methods to serve as an effective and accurate tool to quantify differences in DNA damage at the single cell level and likewise to measure the sensitivity towards DNA damage inducing UV light in cell samples, to ultimately use the results to determine the subjects' susceptibility to cancer.

### DESCRIPTION OF THE PREFERRED EMBODIMENTS

To obtain a specimen or specimens from a subject, peripheral blood is withdrawn by venipuncture and can be used in the following assay method. Preferred protocol describes the use of isolated peripheral blood mononuclear cells (PBMCs), however does not exclude the possibility to use whole blood instead. Staging of tumors was done according to internationally common Overall Stage Grouping which is also referred to as Roman Numeral Staging. This system uses numerals I, II, III, and IV (plus the 0) to describe the progression of cancer. The stages refer to the following:
Stage 0: carcinoma in situ, abnormal cells growing in their normal place ("in situ" from Latin for "in its place").
Stage I: cancers are localized to one part of the body. Stage I cancer can be surgically removed if small enough.
Stage II: cancers are locally advanced. Stage II cancer can be treated by chemotherapy, radiation, or surgery.
Stage III: cancers are also locally advanced. Whether a cancer is designated as Stage II or Stage III can depend on the specific type of cancer; for example, in Hodgkin's Disease, Stage II indicates affected lymph nodes on only one side of the diaphragm, whereas Stage III indicates affected lymph nodes above and below the diaphragm. The specific criteria for Stages II and III therefore differ according to diagnosis. Stage III can be treated by chemotherapy, radiation, or surgery.
Stage IV: cancers have often metastasized, or spread to other organs or throughout the body. Stage IV cancer can be treated by chemotherapy, radiation, or surgery. Despite treatment, a patient's mortality rate can be significantly higher with Stage IV cancer, e.g., the cancer can progress to become terminal.

To quantify DNA damage and DNA damage sensitivity (DDS) respectively, samples are collected before (Control/baseline) and immediately after exposure to UV radiation. This so-called time zero (T0) time point is for practical reasons such as time needed for pipetting, achieved within 15 minutes or less, such as within 12 minutes, 10 minutes, 8 minutes or even 5 minutes.

UV exposure can be either performed by radiation of prepared cell suspension (e.g. PBMC cells resuspended in cell culture medium or PBS in cell culture plate) or directly of samples loaded as spots on plates (glass plates) with agarose, such as 4D Lifeplates. The time point T0 may be more accurate when radiation is performed on spotted samples.

The data shown in Fig. 1 and 2 were obtained by exposing PBMC cells in suspension to UVB radiation. The further procedure, with the exception of radiation, is described below.

Isolated PBMCs were mixed with low melting temperature agarose (LMA in PBS) and kept in a heat block set at 37°C resulting in a final concentration of 0.4% LMA. Directly after mixing, aliquots of the agarose/cell mix (containing 200-400 cells) were applied to a 4D Lifeplate. Those, in agarose embedded cells on a 4D Lifeplate, are herein referred to as spots. The spots are allowed to polymerize for 1-2 min at 4°C and cells were subsequently lysed for 1 hour at 4°C in lysis buffer (NaCl, EDTA, Tris(hydroxymethyl) aminomethane (TRIS)-base, Na-laurylsarcosinate, Triton-X-100). The 4D Lifeplate was shortly washed in double-distilled water (ddH₂O) before being placed into the 4D Lifetank. Therein, the slide was immersed in cold electrophoresis solution (NaOH based,) and undergoes unwinding for 40 min at 4°C. Subsequently, the slide was electrophoresed at 1 V/cm for 30 min and constant cooling at 4°C. After electrophoresis, the Lifeplate was washed twice in neutralization buffer (TRIS-base) for 5 min, rinsed in ddH₂O, before spots were fixed in EtOH for 10 min. Samples were dried at 37°C for 1 hour or overnight at room temperature and DNA was stained by SYBR^{™} Gold (50-240 nM in 30 mM TAE, pH 8.0) for 30 min. After a brief wash with ddH₂O, plates were incubated twice for 30 min in destaining solution (from abcbiopply) and subsequently dried overnight at room temperature or 2 hours at 37°C in the dark.

Slides were imaged with Microscope BX63 (Olympus) with 10x objective and corresponding software Cells Dimension. Scoring of the comets was performed with Andor Komet 7.1.

The inventors used samples derived from the same specimen (same patient) to investigate the influences of the time point or respectively the location of radiation.

Samples derived from the same specimen were irradiated either before or after introduction into agarose gel. The assay protocol varied only in the timepoint of irradiation (V1 = Irradiation of cells before introduction into agarose gel and V2 = Irradiation of cells after introduction into agarose gel). 5 specimen of healthy patients and 5 specimens of patients suffering from lung cancer were tested. The result was independent of the timepoint of radiation, the differences in the raw data were within the previously recorded statistical deviation (with unvaried assay protocol). The diagnostic outcome was the same for samples for each individual patient (see table 1).

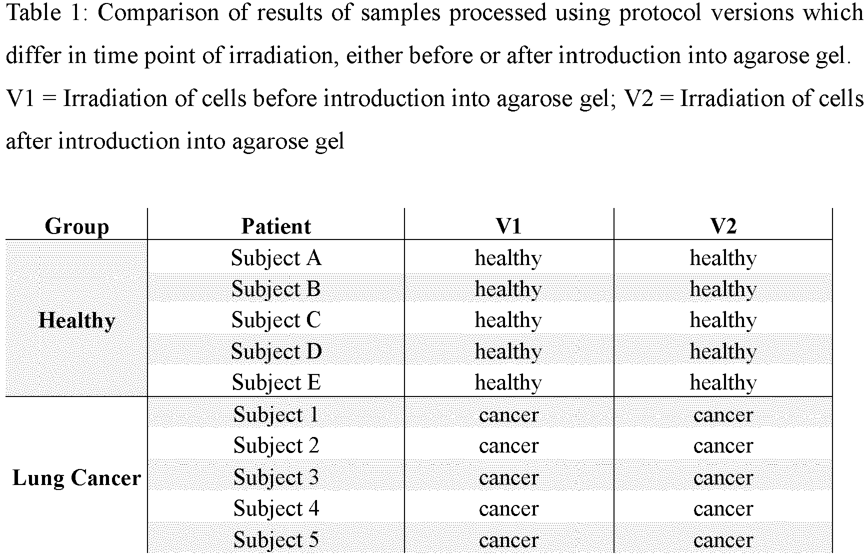

## Claims

1. In vitro or ex vivo use of resistance of genomic DNA against damage by radiation as a biomarker for proliferative diseases and susceptibility thereof, wherein an increased resistance of the genomic DNA is indicative for a proliferative disease and/or increased susceptibility thereof comprising analyzing a biological sample containing cells from a subject to determine the resistance of genomic DNA.

2. Use according to claim 1, wherein the resistance of genomic DNA against damage by radiation is used as a biomarker for breast cancer, lung cancer, prostate cancer and colon cancer or an increased susceptibility for one of these cancers.

3. Use according to claim 1 or 2, wherein the genomic DNA is derived from whole blood cells or peripheral blood mononuclear cells.

4. Use according to any one of claims 1 to 3 to test the effect of chemical compounds, medication, and/or environmental impacts on cancer susceptibility.

5. Use according to any one of claims 1 to 4, wherein at least one further biomarker for cancer is determined.

6. Use according to any one of claims 1 to 5, wherein the DNA damage is determined by evaluation of normalized DNA tail using single cell gel electrophoresis assay.

7. An in vitro or ex-vivo method for determining the resistance of genomic DNA against damage by radiation, comprising:
a) providing a sample containing blood cells,
b) irradiating the blood cells of the sample,
c) performing cell lysis, and
d) determining genomic DNA damage using single cell gel electrophoresis assay, wherein an increased resistance of the genomic DNA is indicative for a proliferative disease and/or increased susceptibility for a proliferative disease.

8. The method according to claim 7, wherein the sample is whole blood or a sample containing isolated peripheral blood cells.

9. The method according to anyone of claims 7 to 8, wherein cell lysis takes place less than 7 minutes after irradiation of the blood cells.

10. The method according to claim 8 or 9, wherein irradiating the peripheral blood cells of the sample is made using UVB radiation.

11. The method according to anyone of claims 7 to 10, wherein determining genomic DNA damage using single cell gel electrophoresis assay comprises calculation and evaluation of DNA tail Percent (DT%).

## Patentansprüche

1. *In vitro* oder *ex vivo* Verwendung der Resistenz genomischer DNA gegen Schädigung durch Strahlung als Biomarker für proliferative Krankheiten und die Anfälligkeit dafür, wobei eine erhöhte Resistenz der genomischen DNA auf eine proliferative Krankheit und/oder eine erhöhte Anfälligkeit dafür hinweist, umfassend die Analyse einer biologischen Probe, die Zellen von einem Subjekt enthält, um die Resistenz der genomischen DNA zu bestimmen.

2. Verwendung nach Anspruch 1, wobei die Resistenz der genomischen DNA gegen Strahlenschäden als Biomarker für Brustkrebs, Lungenkrebs, Prostatakrebs und Dickdarmkrebs oder eine erhöhte Anfälligkeit für eine dieser Krebsarten verwendet wird.

3. Verwendung nach Anspruch 1 oder 2, wobei die genomische DNA aus Vollblutzellen oder mononukleären Zellen des peripheren Blutes gewonnen wird.

4. Verwendung nach einem der Ansprüche 1 bis 3 zur Prüfung der Wirkung von chemischen Verbindungen, Medikamenten und/oder Umwelteinflüssen auf die Krebsanfälligkeit.

5. Verwendung nach einem der Ansprüche 1 bis 4, wobei mindestens ein weiterer Biomarker für Krebs bestimmt wird.

6. Verwendung nach einem der Ansprüche 1 bis 5, wobei der DNA-Schaden durch Auswertung des normalisierten DNA-Schwanzes unter Verwendung eines Einzelzell-Gelelektrophorese-Assays bestimmt wird.

7. *In-vitro-* oder ex-vivo-Verfahren zur Bestimmung der Resistenz genomischer DNA gegen Strahlenschäden, umfassend:
a) Bereitstellung einer Probe, die Blutzellen enthält,
b) Bestrahlung der Blutzellen der Probe,
c) Durchführen einer Zelllyse, und
d) Bestimmung der genomischen DNA-Schädigung unter Verwendung eines Einzelzell-Gelelektrophorese-Assays, wobei eine erhöhte Resistenz der genomischen DNA auf eine proliferative Erkrankung und/oder eine erhöhte Anfälligkeit für eine proliferative Erkrankung hinweist.

8. Verfahren nach Anspruch 7, wobei die Probe Vollblut oder eine Probe ist, die isolierte periphere Blutzellen enthält.

9. Verfahren nach einem der Ansprüche 7 bis 8, wobei die Zelllyse weniger als 7 Minuten nach der Bestrahlung der Blutzellen stattfindet.

10. Verfahren nach Anspruch 8 oder 9, wobei die Bestrahlung der peripheren Blutzellen der Probe mit UVB-Strahlung erfolgt.

11. Verfahren nach einem der Ansprüche 7 bis 10, wobei die Bestimmung des genomischen DNA-Schadens unter Verwendung eines Einzelzell-Gelelektrophorese-Assays die Berechnung und Auswertung des DNA-Schwanzprozentsatzes (DT%) umfasst.

## Revendications

1. Utilisation *in vitro* ou *ex vivo* de la résistance de l'ADN génomique aux dommages causés par les radiations comme biomarqueur des maladies prolifératives et de leur susceptibilité, une résistance accrue de l'ADN génomique indiquant une maladie proliférative et/ou une susceptibilité accrue à celle-ci, comprenant analyse d'un échantillon biologique contenant des cellules provenant d'un sujet pour déterminer la résistance de l'ADN génomique.

2. Utilisation selon la revendication 1, dans laquelle la résistance de l'ADN génomique aux dommages causés par les radiations est utilisée comme biomarqueur du cancer du sein, du cancer du poumon, du cancer de la prostate et du cancer du côlon ou d'une susceptibilité accrue à l'un de ces cancers.

3. Utilisation selon la revendication 1 ou 2, dans laquelle l'ADN génomique est obtenu à partir de cellules de sang total ou de cellules mononucléaires du sang périphérique.

4. Utilisation selon l'une des revendications 1 à 3 pour tester l'effet de composés chimiques, de médicaments et/ou d'influences environnementales sur la prédisposition au cancer.

5. Utilisation selon l'une quelconque des revendications 1 à 4, dans laquelle au moins un autre biomarqueur du cancer est déterminé.

6. Utilisation selon l'une quelconque des revendications 1 à 5, dans laquelle le dommage à l'ADN est déterminé par évaluation de la queue d'ADN normalisée en utilisant un test des "comète" (*Single Cell Gel Electrophoresis*).

7. Méthode *in vitro* ou *ex vivo* pour déterminer la résistance de l'ADN génomique aux dommages causés par les radiations, comprenant :
a) Fourniture d'un échantillon contenant des cellules sanguines,
b) irradiation des cellules sanguines de l'échantillon,
c) effectuer une lyse cellulaire; et
d) détermination des lésions de l'ADN génomique à l'aide d'un test des "comète", dans lequel une résistance accrue de l'ADN génomique indique une maladie proliférative et/ou une susceptibilité accrue à une maladie proliférative.

8. Procédé selon la revendication 7, dans lequel l'échantillon est du sang total ou un échantillon contenant des cellules sanguines périphériques isolées.

9. Procédé selon l'une quelconque des revendications 7 à 8, dans lequel la lyse cellulaire a lieu moins de 7 minutes après l'irradiation des cellules sanguines.

10. Procédé selon la revendication 8 ou 9, dans lequel l'irradiation des cellules sanguines périphériques de l'échantillon est effectuée avec un rayonnement UVB.

11. Procédé selon l'une quelconque des revendications 7 à 10, dans lequel la détermination de les dommages à l'ADN génomique en utilisant un test des "comète" comprend le calcul et l'évaluation du pourcentage de queue d'ADN (DT%).
